# EUROPEAN PATENT APPLICATION

(11) **EP 2 156 825 A1**
(43) Date of publication of application: **24.02.2010**
(21) Application number: 09014697.8
(22) Date of filing: 13.12.2004
(51) Int. Cl.: A61K 9/48, A61K 31/19

(54) **Highly concentrated pourable aqueous solutions of potassium ibuprofen, their preparation and their uses**

(30) Priority: 22.12.2003 US 744545
(62) Divisional of application: 04813842.4
(71) Applicant: Albemarle Corporation, Baton Rouge, LA 70801-1765 (US)
(72) Inventor: Hu, Patrick, C., Baton Rouge, LA 70808 (US); Lambeth, Gregory, H., Baton Rouge, LA 70817 (US); Malcolm, Arcelio, J., Baton Rouge, LA 70810 (US)
(74) Representative: UEXKÜLL & STOLBERG

(57) **Abstract**

Concentrated pourable potassium ibuprofen liquid compositions and their preparation are described. They are comprised of (i) potassium ibuprofen, (ii) water; and (iii) methanol, ethanol, 1-propanol, 2-propanol, 1,1-dimethylethanol, or a mixture of any two or more of them. The amount of dissolved potassium ibuprofen in the composition is in the range of about 60 to about 90 wt%. These compositions are suitable for use in the preparation of pharmaceutical dosage forms such as liquid-filled soft gelatin capsules, syrups, elixirs, suspensions; solid dosage forms such as tablets or caplets; and topically-applied products such as lotions, creams or ointments.

## Description

### TECHNICAL FIELD

This invention relates to new highly concentrated aqueous liquid compositions of what are deemed to be at least partially solvated and/or at least partially ionized and/or at least complexed potassium 2-(4-isobutylphenyl)propionate. Thus it is believed that the compositions may contain at least some potassium cations and 2-(4-isobutylphenyl)-propionate anions. If one were to remove all of the liquid from such compositions, the resultant solids would comprise at least a predominate amount of potassium 2-(4-isobutylphenyl)propionate.

Consequently for convenience, and convenience only, the term "dissolved" in connection with potassium ibuprofen is often used in this document. This term is used in its ordinary meaning to specify that a quantity of potassium ibuprofen has been caused to pass into solution. This term is not intended as a representation that what is in the liquid composition is actually potassium 2-(4-isobutylphenyl)propionate as such. Rather, the term is used to specify that upon the removal of all of the liquid from the concentrated liquid composition, potassium 2-(4-isobutylphenyl)propionate would be present in a solids phase, and that while in the concentrated liquid composition it may be partially or entirely in solvated form and/or in ionized form and/or in complexed form and/or in some other dissolved or soluble form. In short, the amount of the potassium ibuprofen which has dissolved is not in the form of a visually perceivable separate phase such as a solid phase or a gel phase.

### BACKGROUND

U.S. Pat. Nos. 4,859,704 and 4,861,797 describe the formation and use of certain liquid ibuprofen compositions in which there are approximately 25 mg to 400 mg of the ibuprofen composition per 5 mL of the aqueous solution. These values correspond to 0.5 to 8 wt% solutions. A methylcellulose composition such as sodium carboxymethylcellulose with or without sucrose is used as a component in these solutions. According to the latter patent, "the methylcellulose composition renders the ibuprofen soluble in the aqueous medium".

U.S. Pat. No. 6,387,400 describes a way of increasing the concentration of a pharmaceutically active ingredient such as ibuprofen in solutions useful in dosage forms such as in soft gelatin capsules. The process involves use of polyethylene glycol having a molecular weight of about 200 Daltons to about 100,000 Daltons as a solvent for *in situ* formed sodium or potassium salts of the pharmaceutically active ingredient. While solutions with concentrations higher than about 8 wt% are achieved by the process of the foregoing patent, unfortunately the maximum concentration of dissolved potassium ibuprofen shown is about 24.2 wt%, and this was achieved where less than about 50 mole % of the ibuprofen was converted to the potassium salt. Also, complete conversion to either sodium or potassium salts of such ingredient was not achieved inasmuch as attempts to push the reaction to higher concentrations resulted in undesirable reaction with the polyethylene glycol solvent. In order to minimize this undesirable side reaction, elaborate processing steps are employed in the process.

U.S. Pat No. 5,912,011 discloses compositions in which ibuprofen is dissolved in a polyoxyethylene sorbitan fatty acid ester and treated with KOH. From the proportions given in each of Claims 1 and 5 of that patent, the maximum theoretical concentration of dissolved potassium ibuprofen would be 48.4 wt%, in a system containing 48 wt% of the fatty acid ester and 3.6 wt% of water. In the experimental data presented in that patent (Table 1), the concentration of dissolved potassium ibuprofen was 24.2 wt% in a system also composed of 35.2 wt% of ibuprofen, 33.3 wt% of polyethylene glycol (PEG 600), and 7.3 wt% of water.

### BRIEF SUMMARY OF THE INVENTION

This invention provides, *inter alia,* pourable liquid compositions containing at least 50 wt%, preferably at least 60 wt%, more preferably at least about 70 wt%, and still more preferably at least about 80 wt% of dissolved potassium ibuprofen in an aqueous liquid medium that does not require use therein of long chain organic compounds such as polyethylene glycol or polyoxyethylene sorbitan fatty acid ester. A preferred solvent system employed in these pourable liquid compositions is composed of a mixture of water and at least one C₁₋₄ alkanol that is miscible with water at least at 25°C. The phrase "miscible with water at least at 25°C" means that the alkanol(s) when in the liquid state are miscible with water in the liquid state at 25°C and that the alkanol(s) when in the liquid state can also be (but need not be) miscible with water when the alkanol(s) and the water are in the liquid state at temperatures below and/or above 25°C.

In preferred embodiments the new compositions are concentrated pourable potassium ibuprofen liquid compositions comprising (i) potassium ibuprofen, (ii) water; and (iii) ethanol, 1-propanol, or 2-propanol or a mixture of any two or all three of them, wherein the potassium ibuprofen is in dissolved form and wherein the amount of dissolved potassium ibuprofen is in the range of about 60 to about 90 wt%, wherein the total wt% of (i), (ii), and (iii) in the composition is at least about 95 wt%, and wherein the composition is pourable at least at 25°C. More preferably, (iii) consists essentially of ethanol, and most preferably is ethanol.

In another of its embodiments, this invention provides a process which comprises:
a) forming, optionally with agitation, a mixture devoid of yellowish hue from components comprised of ibuprofen, potassium base, water, and optionally alkanol specified hereinafter, wherein the equivalent ratio of potassium base to ibuprofen used in forming said mixture is in the range of about 0.80:1 to about 1.05:1;
b) heating with agitation mixture formed in a) and optionally adding alkanol specified hereinafter, to provide a liquid composition comprised of potassium ibuprofen; and
c) concentrating composition from b) and optionally at least before or during said concentrating, adding alkanol specified hereinafter, such that a pourable concentrated liquid composition is formed that (1) is pourable at least at 25°C, (2) is comprised of potassium ibuprofen, water, and alkanol specified hereinafter, and (3) contains at least about 60 wt% of dissolved potassium ibuprofen;
wherein said alkanol referred to in a), b) and c) hereof is added at least in one of a), b), or c), with the proviso that if said alkanol is added only in c), at least a substantial portion of the alkanol being added is added before or during said concentrating, and wherein said alkanol referred to in a), b), and c) hereof is independently selected from the group consisting of methanol, ethanol, 1-propanol, 2-propanol, 1,1-dimethylethanol, and combinations of any two or more of them. Preferably, the concentrating operation conducted in c), involves removing liquid from the composition from b) by evaporation such as by distillation, flashing, azeotropic distillation, vacuum distillation, or the like, conducted at temperatures of up to about 80°C, although any temperature can be used that does not result in significant color development or decomposition of the product.

Still other embodiments and features of this invention will be still further apparent from the ensuing description and appended claims.

### FURTHER DETAILED DESCRIPTION OF THE INVENTION

Despite their high concentrations, the concentrated liquid compositions of this invention are pourable liquids which can be readily handled and used in processing operations involved in the preparation of pharmaceutical dosage forms. As used herein the term "pourable" means that the liquid composition of this invention, at least when at a temperature of 25°C, can be caused to flow or fall as from one container to another, or into, over, or on something without application of any special force other than gravity as it exists at least at sea level of the Earth. In other words, the phrase "pourable at least at 25°C" as used herein means that the composition when in the liquid state is pourable at 25°C without application of any special force other than gravity as it exists at least at sea level of the Earth, and that the composition when in the liquid state can also be (but need not be) pourable without application of any special force other than gravity as it exists at least at sea level of the Earth, when the composition is at least at one temperature below and/or above 25°C. When used in the production of pharmaceutical preparations, the liquid compositions of this invention in which the alkanol present is ethanol of pharmaceutically-acceptable purity for internal human consumption, are suitable for internal and external usage, whereas the compositions of this invention in which the alkanol present is (A) ethanol not of pharmaceutically-acceptable purity for internal human consumption, and/or (B) methanol, 1-propanol, 2-propanol, 1,1-dimethylethanol, or a combination of any two or more of the foregoing, and with or without ethanol, are not intended as such for direct internal human usage, but rather are for other uses such as in production of external topical skin preparations or the like.

In addition to the foregoing usage considerations, the liquid compositions of this invention fall into two categories. In one such category the liquid composition is a clear single phase liquid composition (in other words, the composition is a transparent solids-free solution having only one liquid phase). In preferred embodiments of this category, the clear single phase liquid composition is stable in the sense that it remains a clear single phase composition for at least 400 hours promptly after preparation when stored in a closed container in the absence of light and at a temperature in the range of 5 to 70°C. In order to evaluate the stability of a highly concentrated liquid composition of this invention to see if it falls into this preferred category, it is not necessary to run tests at various temperatures within the range of 5 to 70°C. A suitable test procedure is to place a sample of a clear single phase promptly after preparation in a closed container in the absence of light in a closed container at a constant temperature of 5°C for 400 hours. If promptly after such storage no separate solid or separate liquid phase in the sample can be seen by the naked eye on viewing the composition under light in the visible wavelength range, the sample is deemed to be stable. It is not necessary to perform the same storage tests at any temperature above 5°C because if the sample passes the foregoing test at 5°C, experience has shown that it will pass the test at higher temperatures within the range of 5 to 70°C.

Thus clear single phase liquid compositions of this invention in which the alkanol present is ethanol of pharmaceutically-acceptable purity for internal human consumption (especially when they are also stable compositions as discussed above) are highly suitable for use in the preparation of any of a variety of pharmaceutical dosage forms, such as filled soft capsules, as well as syrups, elixirs, suspensions, and other pharmaceutical dosage forms including solid dosage forms such as tablets, caplets, and filled hard gelatin capsules. Because they are stable for at least 400 hours promptly after preparation, such compositions are likely to remain in the same stable condition for even longer periods of time as long as they are not subjected to destructive high temperatures or some other condition adverse to stability.

The clear single phase liquid compositions of this invention in which the alkanol present is one or more water miscible C₁₋₄ alkanols other than ethanol of pharmaceutically-acceptable purity for internal human consumption (especially when they are also stable compositions as discussed above) are highly suitable for use in the preparation of various pharmaceutical dosage forms for external administration, such as salves, creams, ointments, lotions, topically applied liquids, and like forms.

As between (1) the clear single phase liquid compositions of this invention in which the alkanol present is solely ethanol of pharmaceutically-acceptable purity for internal human consumption, especially when the compositions are also stable compositions and (2) the clear single phase liquid compositions of this invention in which at least one water miscible C₁₋₄ alkanol is other than ethanol of pharmaceutically-acceptable purity for internal human consumption, especially when the compositions are also stable compositions, the compositions of (1) are preferred because of their wider fields of use.

The second such category of the liquid compositions of this invention are those which are hazy, cloudy, or turbid in appearance and/or contain particles that can be removed by suitable physical techniques such as vacuum filtration or centrifugation at up to about 80°C, and which nevertheless contain at least 60 wt% of dissolved potassium ibuprofen. In cases where the haze and/or the particles cannot be removed, such compositions, though less preferred, are useful for preparation of pharmaceutical dosage forms such as products for external human application such as suspensions, salves, creams, and lotions. If the alkanol present in such compositions is solely ethanol of pharmaceutically-acceptable purity, the composition may also be used in the preparation of suspensions for internal human administration, if desired.

The pourable concentrated liquid compositions of this invention preferably contain at least about 70 wt% of dissolved potassium ibuprofen, more preferably at least about 80 wt% of dissolved potassium ibuprofen, and most preferably at least about 85 wt% of dissolved potassium ibuprofen.

Use of any ingredient other than potassium ibuprofen, water and methanol, ethanol, 1-propanol, 2-propanol, 1,1-dimethylethanol, or a mixture of any two or more of them (hereinafter sometimes referred to collectively as "water miscible C₁₋₄ alkanols") is not required to produce the concentrated liquid compositions of this invention. Thus components such as carboxymethylcellulose composition and/or sucrose solubilizing agents, glycols such as polyethylene glycol, or polyoxyethylene sorbitan fatty acid ester and/or propylene glycol are not required. However, if desired, small amounts of one or more additional organic liquid additives such as glycerol, polyethylene glycol, polyoxyethylene sorbitan fatty acid ester, and/or propylene glycol can be included in the compositions of this invention, with the provisos that (A) when the concentration of the dissolved potassium ibuprofen in the composition of this invention is in the range of about 60 to about 70 wt%, the amount of such additive(s) can be up to but no more than about 5 wt% of the total weight of the composition; (B) when the concentration of the dissolved potassium ibuprofen in the composition of this invention is in the range of about 70 to about 80 wt%, the amount of such additive(s) can be up to but no more than about 3 wt% of the total weight of the composition; and (C) when the concentration of the dissolved potassium ibuprofen in the composition of this invention is in the range of about 80 to about 90 wt%, the amount of such additive(s) can be up to but no more than about 2 wt% based on the total weight of the composition. The preferred compositions of this invention contain no organic solvent or organic solubilizing agent other than one or more water miscible C₁₋₄ alkanol(s). Of the water miscible C₁₋₄ alkanols, ethanol, 1-propanol, 2-propanol or a mixture of any two or all three of them are preferred alkanols for use in the practice of this invention. More preferred is ethanol, and most preferred is ethanol of pharmaceutically-acceptable purity.

In one of its embodiments this invention provides a concentrated pourable stable potassium ibuprofen liquid composition comprising (i) potassium ibuprofen, (ii) water, and (iii) ethanol, preferably ethanol of pharmaceutically-acceptable purity, wherein the amount of dissolved potassium ibuprofen is in the range of about 60 to about 90 wt%, wherein the total wt% of (i), (ii), and (iii) in the composition is at least about 95 wt%, and wherein the weight ratio of ethanol to water is in the range of about 0.25:1 to about 4.0:1. More preferably, the dissolved potassium ibuprofen concentration in the composition is in the range of about 70 to about 90 wt% and the ethanol to water weight ratio is in the range of about 0.30:1 to about 1:1. Still more preferably, the dissolved potassium ibuprofen concentration in the composition is in the range of about 80 to about 90 wt% and the ethanol to water weight ratio is in the range of about 0.30:1 to about 0.80:1. Even more preferably, the dissolved potassium ibuprofen concentration in the composition is in the range of about 85 to about 90 wt% and the ethanol to water weight ratio is in the range of about 0.30:1 to about 0.60:1. In the most preferred compositions of this embodiment of the invention, except for the optional presence of an excess amount (where the equivalent ratio of potassium base to ibuprofen is in the range of about 0.80:1 to about 1.05:1, but is not 1:1) of unreacted ibuprofen or unreacted potassium base, and except for the optional presence of trace amounts of one or more impurities and/or manufacturing by-products, the composition is composed only of (i), (ii), and (iii).

In another embodiment of this invention there is provided a concentrated pourable stable potassium ibuprofen liquid composition adapted for external pharmaceutical uses *e.g.,* as or in a topically-applied pharmaceutical preparation, said liquid composition comprising (i) potassium ibuprofen, (ii) water; and (iii) one or more water miscible C₁₋₄ alkanols, wherein the amount of dissolved potassium ibuprofen is in the range of about 60 to about 90 wt%, wherein the total wt% of (i), (ii), and (iii) in the composition is at least about 95 wt%, and wherein the weight ratio of (iii) to water is in the range of about 0.25:1 to about 4:1. In the most preferred compositions of this embodiment of the invention, except for the optional presence of an excess amount of unreacted ibuprofen or unreacted potassium base (where the equivalent ratio of potassium base to ibuprofen is in the range of about 0.80:1 to about 1.05:1, but is not 1:1), and except for the optional presence of trace amounts of one or more impurities and/or manufacturing by-products, the composition is composed only of (i), (ii), and (iii). In these embodiments of the invention, it is not necessary to employ ethanol of pharmaceutically-acceptable purity, and thus compositions devoid of ethanol pharmaceutically-acceptable purity are less expensive.

A feature of this invention is that the high concentrations characterizing the compositions of this invention is achieved using potassium ibuprofen in which at least 80% of the ibuprofen has been neutralized by potassium base. It is thus possible to modify the pH of the resultant concentrated composition such that it is more amenable for use in capsules made of various materials. In this connection, as U.S. Pat. No. 5,071,643 states in connection with fill liquids for use in softgel capsules, that the pH of the fill liquid should not be below 2.5 or above 7.5, and that at pH's below 2.5, the gelatin is hydrolyzed causing leakage.

The above highly concentrated ethanol-containing liquid compositions of this invention (*i*.*e*., those that are devoid of methanol, 1-propanol, 2-propanol, 1,1-dimethylethanol, and where the ethanol is of pharmaceutically-acceptable purity) are especially useful in a variety of pharmaceutical applications in that (i) the compositions can be used as such in filling capsules, including soft capsules, (ii) the compositions can be used in formulating liquid dosage forms to be used in filling capsules, including soft capsules, and (iii) the compositions can be used in the manufacture of syrups, suspensions, elixers, and other liquid dosage forms for internal or external administration. In addition, these ethanol-containing compositions of this invention devoid of any water miscible C₁₋₄ alkanol other than ethanol of pharmaceutically-acceptable purity, can be used in the formation of solid pharmaceutical dosage forms such as by absorption onto or adsorption into suitable excipients or carriers that are in solid form. Such dosage forms devoid of any water miscible C₁₋₄ alkanol other than ethanol of pharmaceutically-acceptable purity, are suitable for internal or external application provided no other component is included which would preclude internal administration of the dosage form. As noted above, when used for production of pharmaceutical preparations, the highly concentrated water miscible C₁₋₄ alkanols other than ethanol of pharmaceutically-acceptable purity, are as such intended for external uses only, such as in topically-applied skin preparations. If all of the methanol, 1-propanol, 2-propanol, or 1,1-dimethylethanol, or mixture of any two or more thereof present in the composition is subsequently replaced by one or more pharmaceutically-acceptable liquids and if all ethanol present in the composition is of pharmaceutically-acceptable purity, then the resultant composition can be used for internal administration.

This invention also provides novel process technology for producing the above-described highly concentrated and eminently useful compositions in yields as high as 100%. For example, in one embodiment of this invention, a concentrated potassium ibuprofen liquid composition of this invention is formed by use of a process which comprises:
a) forming, optionally with agitation, a mixture devoid of yellowish hue from components comprised of ibuprofen, potassium base, water, and optionally alkanol specified hereinafter, wherein the equivalent ratio of potassium base to ibuprofen used in forming said mixture is in the range of about 0.80:1 to about 1.05:1;
b) heating with agitation mixture formed in a) and optionally adding alkanol specified hereinafter, to provide a liquid composition comprised of potassium ibuprofen; and
c) concentrating composition from b) and optionally at least before or during said concentrating, adding alkanol specified hereinafter, such that a pourable concentrated liquid composition is formed that (1) is pourable at 25°C, (2) is comprised of potassium ibuprofen, water, and alkanol specified hereinafter, and (3) contains at least about 60 wt% of dissolved potassium ibuprofen;
wherein said alkanol referred to in a), b) and c) hereof is added at least in one of a), b), or c), with the proviso that if said alkanol is added only in c), at least a substantial portion of the alkanol being added is added before or during said concentrating, and wherein said alkanol referred to in a), b), and c) hereof is at independently selected from the group consisting of methanol, ethanol, 1-propanol, 2-propanol, 1,1-dimethylethanol, and combinations of any two or more of them (i.e., such alkanol is at least one water miscible C₁₋₄ alkanol). When the alkanol is added only in c), at least a substantial portion of the alkanol being added is added before or during said concentrating so that there is enough alkanol present to result in the formation of a pourable concentrated liquid composition of this invention. In such cases some additional alkanol can also be added after the concentrating operation has been completed.

In a preferred embodiment, in conducting the above process an inert organic solvent that forms an azeotrope with water and that is other than methanol, ethanol, 1-propanol, 2-propanol, 1,1-dimethylethanol or a combination of any two or more of them (whichever C₁₋₄ alkanol is used), is added (A) at least in a) to said mixture in a), or (B) at least in b) to said mixture in b), or (C) at least in c) to the composition from b) before or during said concentrating. Preferably, the concentrating is conducted such that the concentrated liquid composition formed pursuant to this invention is substantially free of the inert organic solvent. By "substantially free" of the inert organic solvent is meant that trace amounts or even more than trace amounts may be present in the final composition of this invention provided that the residual amount of inert organic solvent is a pharmaceutically-acceptable amount in the particular pharmaceutical operation to which the final composition will be put. Generally speaking, the lower the amount of residual organic solvent, if any, in the final composition of this invention, the better. It is important to note that the concentrating in c) need not be conducted in one operation. If desired, the concentrating operation of c) can be conducted in stages and at different locations and/or at different times to produce a composition of this invention. The composition of the final compositions of this invention are as described elsewhere herein.

For convenience, the term "azeotropic solvent" is sometimes used hereinafter to refer to the inert organic solvent that is other than one or more water miscible C₁₋₄ alkanols, and that forms an azeotrope with water that boils below about 80°C at atmospheric or subatmospheric pressure.

When conducting any process of this invention it is preferred to produce a product in which the dissolved potassium ibuprofen concentration in the composition is in the range of about 70 to about 90 wt% and the weight ratio of the water miscible C₁₋₄ alkanol(s) to water is in the range of about 0.30:1 to about 1:1. Still more preferably, the dissolved potassium ibuprofen concentration in the composition is in the range of about 80 to about 90 wt% and the weight ratio of the water miscible C₁₋₄ alkanol(s) to water is in the range of about 0.30:1 to about 0.80:1. Even more preferably, the dissolved potassium ibuprofen concentration in the composition is in the range of about 85 to about 90 wt% and the weight ratio of the water miscible C₁₋₄ alkanol(s), most preferably ethanol, to water is in the range of about 0.30:1 to about 0.60:1. As will be seen from the Examples hereinafter, pourable liquid compositions of this invention have been formed containing as high as about 89.8 wt% of dissolved potassium ibuprofen in a mixture of water and ethanol.

Thus, the amount of at least one water miscible C₁₋₄ alkanol added in one or more in steps a), b), and c) above is such that the concentrated liquid composition of this invention formed in the process achieves a water miscible C₁₋₄ alkanol to water weight ratio specified herein. It is to be noted that some water miscible C₁₋₄ alkanol is removed when conducting the concentration operation in step c) of the process. Therefore if water miscible C₁₋₄ alkanol is added prior to step c), the amount of water miscible C₁₋₄ alkanol present in the mixture formed from ibuprofen, potassium base, water, and at least one water miscible C₁₋₄ alkanol (and optionally azeotropic solvent) can result in the water miscible C₁₋₄ alkanol to water weight ratio prior to step c) being higher than a ratio specified herein. For example, if all of the water miscible C₁₋₄ alkanol is added prior to step c), the amount of one or more water miscible C₁₋₄ alkanols added can be as high as about 10 parts by weight for each 1 part by weight of water present.

Preferably, some or all of at least one water miscible C₁₋₄ alkanol is added in step a) above. This is advantageous in that in addition to increasing the solubility of potassium ibuprofen in water, the water miscible C₁₋₄ alkanol(s) used helps in controlling the amount of foam formed during the acid-base reaction in a). Also, the presence of at least one water miscible C₁₋₄ alkanol in step a) above facilitates dissolution of ibuprofen and thereby expedites the formation of potassium ibuprofen *in situ.* Moreover, in conducting step a) it is desirable to avoid using excessive amounts of water as use of smaller amounts of water will reduce the amount of water to be removed in step c). However, in using small amounts of water, the reaction mixture in step a) becomes difficult to agitate and thus the addition of at least one water miscible C₁₋₄ alkanol in step a) is of further advantage in that it facilitates agitation through viscosity reduction.

Use of at least one water miscible C₁₋₄ alkanol in any of steps a), b), and/or c) provides still another advantage to the process. In particular, because a water miscible C₁₋₄ alkanol forms an azeotrope with water, this enables the water to be removed more efficiently in step c). Still further, the azeotrope of water and a water miscible C₁₋₄ alkanol can be recycled to step a) and thereby provide more efficient utilization of the water miscible C₁₋₄ alkanol(s) used.

In step a) the components ibuprofen, potassium base, water, and if used in step a), at least one water miscible C₁₋₄ alkanol, can be brought together in various ways and sequences. For example these components can be charged to a vessel individually in any order, or any two or more of them can be individually charged concurrently, again in any sequence when also charging one or more other component(s). Alternatively, the charging can involve use of various preformed subcombinations. Thus, for example, the total amount of ibuprofen and potassium base can be provided by charging an appropriate amount of preformed potassium ibuprofen powder or particles instead of potassium base and ibuprofen. If desired, the total amount of ibuprofen and potassium base can be provided by charging some potassium base and/or ibuprofen before, at the same time, and/or after such preformed potassium ibuprofen is charged. Similarly, if at least one water miscible C₁₋₄ alkanol is being used in step a), the water and the water miscible C₁₋₄ alkanol(s) can be premixed and added as such, optionally with separate addition of more of either one or both of them. Still other ways of getting these components into the vessel exist and can be used. Although there is nothing critical about how the components are brought together in step a) of the process, it is preferable to add the potassium base and water incrementally, either separately or in combination, or both, to the ibuprofen while agitating the reaction mixture.

In conducting step a) of the process any potassium base that can form a potassium salt of ibuprofen can be used. However for pharmaceutical uses the potassium base employed in the process must not contribute pharmaceutically unacceptable species. Non-limiting examples of suitable bases include for example potassium carbonate, potassium bicarbonate, potassium hydroxide, potassium silicate, potassium oxide, one or more water-soluble potassium salts of inorganic acids of phosphorus, and mixtures of any two or more of the foregoing. Use of potassium carbonate or potassium hydroxide or a combination of these as the base is preferred.

Typically the proportion of potassium base used in step a) will be such that the equivalent ratio of ibuprofen to the potassium base is in the range of about 0.80:1 to about 1.05:1, preferably in the range of about 0.90:1 1 to about 1:1, and most preferably in the range of about 0.95:1 to about 0.98:1. It will of course be understood that in the reaction with ibuprofen, 1 equivalent of a potassium base having 1 atom of potassium per molecule (e.g., KOH) is 1 mole thereof. On the other hand, 1 equivalent of a potassium base having 2 atoms of potassium per molecule, such as potassium carbonate (K₂CO₃), is 0.5 mole thereof. It will be appreciated therefore that the potassium ibuprofen in the compositions of this invention has a mole ratio of potassium to ibuprofen in the range of about 0.80:1 to about 1.05:1 and preferably in the range of about 0.90:1 1 to about 1:1. Most preferably, the mole ratio of potassium to ibuprofen in the compositions of this invention is in the range of about 0.95:1 to about 0.98:1.

According to strict chemical theory, potassium 2-(4-isobutylphenyl)propionate is made up of 1 atom of potassium per molecule of ibuprofen which has been neutralized by the potassium base used. But if the reaction does not go to completion, there can be some unreacted potassium base or unreacted ibuprofen present in the potassium ibuprofen compositions of this invention. Moreover, there can be some free potassium base or some free ibuprofen present in the potassium ibuprofen compositions of this invention because of the use of the ibuprofen and the potassium base in an equivalent ratio specified in the immediately preceding paragraph in which one or the other such reactant is present in an excess over the precise potassium:ibuprofen stoichiometric ratio of 1:1. Therefore for convenience, when reference is made herein, including the claims, to the potassium ibuprofen in a composition of this invention having a mole ratio of potassium to ibuprofen in the range of, say, about 0.80:1 to about 1.05:1, this means that if a sample of the concentrated clear, or hazy, cloudy, or turbid, potassium ibuprofen liquid composition were to be analyzed, the analysis, if properly conducted, would indicate that the ratio of potassium to ibuprofen in the composition is in that specified range. The use of such ratio is not to be interpreted to refer to any change in the actual molecular structure of potassium 2-(4-isobutylphenyl)propionate when in solid form. Rather, the use of such ratio is to be understood to mean that the potassium 2-(4-isobutylphenyl)propionate is in whatever chemical form(s) it exists while in the liquid composition of this invention and that if all liquids were removed from the composition, the dry residue would contain potassium 2-(4-isobutylphenyl)propionate and optionally at least some free ibuprofen or some free potassium base.

In conducting step a) it is desirable to minimize the amount of water used. Thus, although the weight ratio of water to ibuprofen can be very large, e.g., 1:0.1 or more, the amount of water to be removed in step c) of the process would be unnecessarily large. Thus as a practical matter, one should employ an amount of water that does not require excessive water removal after completion of the reaction. In selecting the weight ratio of water to ibuprofen for use in any given situation, one should also take into consideration the water solubility of the potassium base being employed. Thus, when using such bases as potassium hydroxide and potassium oxide, which have higher water solubilities than, say, potassium bicarbonate, the amount of water used can be smaller than when using a less soluble base such as potassium bicarbonate. Generally speaking, it is convenient to use weight ratios of water to ibuprofen in the range of about 0.2:1 to about 0.8:1 and preferably in the range of about 0.25:1 to about 0.40:1.

Step a) of the process is typically conducted at ambient room temperature but can be conducted at a reduced temperature (e.g., down to about 10°C), or at an elevated temperature (e.g., up to about 60°C), if desired. In short, any temperature at which the components can be mixed without undue difficulty can be employed in step a).

In step b) of the process, the mixture is heated under conditions that do not result in visually-observable color formation in the product composition being formed. The temperature to which the mixture is heated can be anywhere within the range of from above room temperature up to about 80°C. Since the time of the operation is inversely proportional to the temperature, it is desirable to heat the mixture to a temperature of at least about 40°C although the operation can be conducted at temperatures between room temperature and 40°C, for longer periods of time. Thus, as long as the temperature does not exceed about 80°C, the temperature at which the operation is conducted is primarily a matter of choice.

In conducting step b) exposure to free oxygen is kept to a minimum, especially when operating at temperatures in the range of about 70 to about 80°C. Thus it is desirable to operate under an inert atmosphere such as nitrogen, argon, neon, krypton, or the like, or to operate under partial vacuum. Even at lower temperatures, e.g., in the range of about 40 to about 70°C it is prudent to operate in a closed system having a relatively small head space, under an inert atmosphere, or under a partial vacuum, but this is not essential so long as color development in the composition does not occur. It can be seen, therefore, that step b) is desirably conducted in a substantially oxygen-free environment. By "substantially oxygen-free" is meant that the system contains either no free oxygen or contains an amount of free oxygen that does not result in visually-observable color formation in the product composition being formed. While on the subject of conditions used in step b), it is worth noting that when employing potassium carbonate (or any other less soluble potassium base) and employing water to potassium carbonate weight ratios of less than 1, it is desirable to use temperatures in the range of about 60 to about 80°C to facilitate dissolution of the potassium carbonate in the relatively small quantity of aqueous solvent medium employed.

In any or all of steps a), b), and c) above, an azeotropic solvent can be added so that on concentrating the resulting composition in step c) to form a concentrated liquid composition devoid of such azeotropic solvent. Non-limiting examples of suitable azeotropic solvents include toluene, n-hexane, n-heptane, ethanol, ethylbenzene, ethyl acetate, or the like. At present, toluene and n-hexane are the preferred azeotropic solvents.

To effect concentration of the composition in step c), all or a portion of the composition formed in b) is subjected to flashing or distillation to remove some water and water miscible C₁₋₄ alkanol(s) from the composition. Evaporation of these solvents can be conducted using conventional equipment such as a wiped film evaporator or spray dryer. The concentration operation of step c) is typically conducted at reduced pressure and at elevated temperatures sufficient to remove some of the water and the water miscible C₁₋₄ alkanol(s) and thereby produce the concentrated pourable potassium ibuprofen liquid composition containing at least about 60 wt% of dissolved potassium ibuprofen and as high as about 90 wt% of dissolved potassium ibuprofen in a liquid medium composed chiefly of water together with at least one water miscible C₁₋₄ alkanol. In many cases the pourable compositions of this invention formed in c) are clear and homogeneous. However, in some cases the pourable compositions of this invention formed in c) are turbid especially when the liquid is maintained at or below ambient room temperature. By "turbid" is meant that the composition is hazy or cloudy in appearance and may contain particles or sediment that can be removed by ordinary physical solids/liquid separation techniques such as centrifugation or decantation. As between the pourable clear homogeneous compositions of this invention and the pourable turbid compositions of this invention, the pourable clear homogeneous compositions of this invention are preferred. If desired, it is often possible to convert a pourable turbid composition of this invention into a pourable clear homogeneous composition of this invention by use of ordinary physical solids/liquid separation techniques such as centrifugation or decantation or by adjusting the alkanol to water ratio of the composition.

Another way of preparing the concentrated liquid compositions of this invention is to procure or preform the potassium ibuprofen for use as a starting material. Such potassium ibuprofen may contain excess unreacted ibuprofen or unreacted potassium base but should have a mole ratio of ibuprofen moiety to potassium in the range of about 0.80:1 to about 1.05:1, preferably in the range of about 0.90:1 to about 1:1, and most preferably in the range of about 0.95:1 to about 0.98:1. In this mode of operation, the potassium ibuprofen is mixed with water and an excess amount of at least one water miscible C₁₋₄ alkanol, and the resultant mixture is heated, preferably with agitation, to evaporate by flashing, distillation, vacuum distillation, or the like, an alkanol-water mixture and thereby form in one or more stages a composition of this invention. In this mode of operation use of an azeotropic solvent is unnecessary.

If desired, small amounts (*e*.*g*., up to about 5 wt%) of pharmaceutically-acceptable excipients, preservatives, sweeteners, or the like can be included in the compositions of this invention. Examples of such materials include glycerol, methylparaben, and sorbitol. Preferably, however, the compositions of this invention are prepared from nothing other than water, at least one water miscible C₁₋₄ alkanol, (most preferably ethanol only), ibuprofen, and potassium base (or preformed potassium ibuprofen as a partial or total replacement for ibuprofen and potassium base). Thus most preferably, the compositions of this invention contain in addition to potassium ibuprofen (in whatever chemical form or forms it exists while in a concentrated pourable potassium ibuprofen liquid composition of this invention), only water, at least one water miscible C₁₋₄ alkanol, and optionally unreacted ibuprofen or potassium base, and possibly impurities that may be in the materials used to prepare the compositions of this invention and/or that result from the manufacturing operations employed in forming the compositions of this invention.

Methods of providing concentrated pourable potassium ibuprofen liquid compositions in individual pharmaceutical dosage forms constitute still additional embodiments of this invention. In general, such methods comprise encapsulating individual pharmaceutical dosage portions of such composition within gelatin shells or soft shells made from other suitable substances such as a composition comprised of a modified starch and iota-carrageenan. This can be accomplished by various techniques such as encapsulation techniques of types such as described for example in U.S. Pat. Nos. 2,234,479; 5,209,978; 6,340,473; 6,569,363; 6,589,536; Published International Application WO 98/42294 published October 1, 1998; The Theory and Practice of Industrial Pharmacy, Leon Lachman, Herbert A. Lieberman and Joseph L. Kanig, editors, 3rd Edition, 1986, Lea & Febiger, Philadelphia, PA, Publishers**,** and Ebert, Soft Elastic Gelatin Capsules: A Unique Dosage Form, Pharmaceutical Technology, October 1977. The encapsulation techniques described in such references are incorporated herein by reference as descriptive of techniques that can be utilized to form individual pharmaceutical dosage forms of this invention. Thus use can be made of rotary die encapsulation processes, reciprocating die encapsulation processes, concentric cylinder processes, and film-enrobing processes all of which are known in the art. Of such encapsulation processes, the rotary die process is presently preferred for use in the practice of this embodiment of the invention.

Machinery for producing soft capsules of the type herein involved, is available from various manufacturers. For example, one may utilize model VSG-172A Softgel manufacturing line produced by Vanguard Pharmaceutical Machinery, Inc., USA which includes an advanced design rotary die encapsulator as well as various associated equipment. Other commercially available equipment for producing soft capsules are models CS-M3 and Model CS-J1 Soft Gel machines available from Daesung Corporation, Daesung B/D 3F, 9-1, Yangpyong 1-dong, Youngdeungpo-ku, Seoul, 150-101 Korea; and Models SGM-1000 and SGM-2000 machines available from Technophar Equipment and Service Limited, 1370 Argyl Road, Windsor, Ontario, N8Y 3K7, Canada.

Thus there is provided pursuant to an embodiment of this invention, a method of providing potassium ibuprofen in individual pharmaceutical dosage forms, which method comprises encapsulating individual pharmaceutical dosage portions of a concentrated pourable potassium ibuprofen liquid composition of this invention within gelatin shells. Preferably in conducting this method the gelatin shells are formed by sealing ribbons of gelatin together around said individual dosage portions of the concentrated pourable potassium ibuprofen liquid composition to thereby encapsulate said dosage portions. More preferably the gelatin shells formed in these methods are soft gelatin shells. In particularly preferred embodiments, the foregoing methods are practiced using a concentrated pourable potassium ibuprofen liquid composition of this invention wherein the dissolved potassium ibuprofen in said composition has an analyzable mole ratio of potassium to ibuprofen in the range of about 0.95:1 to about 0.98:1, and most especially wherein the amount of dissolved potassium ibuprofen in such composition is in the range of about 80 to about 90 wt%, and the ethanol and the water are in a weight ratio of ethanol to water in the range of about 0.30:1 1 to about 0.80:1.

Also provided by this invention is an article of manufacture which comprises at least one pharmaceutical capsule defining an interior enclosed space, said capsule being sized and shaped for oral administration, and containing within said space a quantity of a concentrated pourable potassium ibuprofen liquid composition comprising (i) potassium ibuprofen, (ii) water; and (iii) ethanol, wherein the composition contains an amount of dissolved potassium ibuprofen in the range of about 60 to about 90 wt%, wherein the total wt% of (i), (ii), and (iii) in the composition is at least about 95 wt%, and wherein the composition is pourable at 25°C. Preferably such article is a soft gelatin capsule, especially a seamless soft gelatin capsule, or a soft capsule that comprises a modified starch and iota-carrageenan (see for example U.S. Pat. No. 6,340,473. It these articles the dissolved potassium ibuprofen in the encapsulated composition preferably has an analyzable mole ratio of potassium to ibuprofen in the range of about 0.95:1 to about 0.98:1, especially wherein the amount of dissolved potassium ibuprofen in such composition is in the range of about 80 to about 90 wt%, and wherein the ethanol and the water are in a weight ratio of ethanol to water in the range of about 0.30:1 to about 0.80:1.

The term "analyzable mole ratio of potassium to ibuprofen" as used herein including the claims means that if the liquid composition being referred to is subjected to analysis, the results of the analysis will indicate that the composition has a mole ratio of potassium to ibuprofen that is in the range specified. This term does not mean that the composition must be analyzed; rather it means only that if one elects to analyze the composition, the analytical results will indicate that the range of mole ratios specified has been complied with. Similarly this term does not constitute, by implication or otherwise, a description of the chemical form in which the potassium ibuprofen exists while dissolved in the composition. As pointed out hereinabove, the potassium ibuprofen can be in whatever chemical form or forms it exists during the time it remains dissolved in the composition.

Another embodiment of this invention is a pharmaceutical product formed from at least one component comprised of a liquid composition according this invention. The pharmaceutical products of this invention may be in liquid, solid, or a combination of liquid and solid form (e.g., gel encapsulation). Still another embodiment of this invention is a process of administering, to a mammal exhibiting at least one symptom responsive to analgesic treatment (e.g., pain, fever, swelling, *etc.*), a pharmaceutically effective amount of a pharmaceutical product of this invention. The pharmaceutical product of this invention may include additional components typically present in conventional liquid, solid and/or encapsulated pharmaceutical products. Suitable non-limiting examples of such other components include viscosity modifiers, flavoring, sweetners, colorants, stabilizers or other preservatives and the like. The product is typically administered orally. When administering the product, the pharmaceutically effective amount employed may vary, but should be sufficient to trigger a discernable analgesic symptomatic response. In most cases, the pharmaceutically effective amount will be an amount sufficient to provide to the mammal an *in situ* amount of an acid form of ibuprofen in the range of about 20 to about 500 mg, since the potassium ibuprofen will normally convert to the acid form after administration.

The practice and advantages of this invention are illustrated by the following examples in which all percentages are by weight.

### EXAMPLE 1

Potassium carbonate (69.1 grams) is weighed into a stainless steel mixing bowl. To this is added 75 grams of deionized water and the bowl is placed under a homogenizer (Dispermat CV by VMA-Getzmann) equipped with a high shear dispersing impeller and mixed therewith. After the K₂CO₃ is substantially dissolved in the water, 206 grams of ibuprofen powder (Albemarle Corporation) is added to the mixing bowl while continuing the mixing. Then 80 grams of anhydrous ethanol is added to the mixture and the mixing is continued for another 30 minutes. After discontinuing the mixing, another 220 grams of anhydrous ethanol is used to rinse the mixing bowl and the contents of the bowl are quantitatively transferred to a 2-liter round bottom flask. The flask is installed in a Rotavapor RE11 (manufactured by Buchi) and heated to 70°C under rotation, until the blend becomes clear (or at least slightly hazy or cloudy). After the contents of the flask become clear (or slightly hazy or cloudy), vacuum provided by an aspirator pump is used to remove solvents (water and ethanol). After a substantial amount of the solvents have been removed, normally a clear stable potassium ibuprofen liquid composition is obtained which remains clear and stable after cooling to room temperature, and after storage for 400 hours in a closed container in the absence of light at a constant temperature of 5°C. NMR analysis shows that a sample of the liquid composition produced in this manner contains about 83.9 wt% of potassium ibuprofen, about 9.7 wt% of water, and about 6.3 wt% of ethanol. The ethanol to water weight ratio is about 0.65:1.

### EXAMPLE 2

The procedure of Example 1 is repeated in substantially the same manner. Normally, a clear, stable liquid composition is produced which is indicated by NMR analysis to contain about 83.2 wt% of potassium ibuprofen, about 10.8 wt% of water, and about 6.0 wt% of ethanol. In this case the ethanol to water weight ratio is about 0.56:1.

### EXAMPLE 3

The procedure of Example 1 is repeated in substantially the same manner except that the blending is conducted in a Hobart mixer and the amount of solvents removed by the aspirator pump is somewhat less than in Example 1. Normally, a clear stable liquid composition is formed which by NMR analysis is indicated to contain about 81.7 wt% of potassium ibuprofen, about 11.3 wt% of water, and about 7.1 wt% of ethanol. The ethanol to water weight ratio is about 0.63:1.

### EXAMPLE 4

Potassium carbonate (69.1 grams) is weighed into a Hobart stainless steel mixing bowl. To this is added 80 grams of deionized water and mixture is subjected to mixing with a Hobart mixer. After the K₂CO₃ is substantially dissolved in the water, 206 grams of ibuprofen powder (Albemarle Corporation) is added to the mixing bowl while continuing the mixing. Then 80 grams of anhydrous ethanol is added to the mixture and the mixing is continued for another 30 minutes. After discontinuing the mixing, another 175 grams of anhydrous ethanol is used to rinse the mixing bowl and the contents of the bowl are quantitatively transferred to a 2-liter round bottom flask. The flask is installed in a Rotavapor RE1 (manufactured by Buchi) and heated to 70°C under rotation, until the blend becomes clear. After the contents of the flask become clear, vacuum provided by an aspirator pump is used to remove solvents (water and ethanol). After a substantial amount of the solvents have been removed, normally a clear, stable potassium ibuprofen liquid composition is obtained which remains clear and stable after cooling to room temperature, and after storage for 400 hours in a closed container in the absence of light at a constant temperature of 5°C. NMR analysis shows that a sample of the liquid composition produced in this manner contains about 82.8 wt% of potassium ibuprofen, about 12.2 wt% of water, and about 5.0 wt% of ethanol. The ethanol to water weight ratio is about 0.41:1.

### EXAMPLE 5

A solution is formed by dissolving 69.1 grams of potassium carbonate in 75 grams of deionized water in a 300 mL beaker. In the stainless steel bowl of a Hobart mixer, a solution is formed from 206 grams of ibuprofen and 300 grams of anhydrous ethanol. The contents of the beaker are poured into the stainless steel bowl and the resultant mixture is subjected to blending with the Hobart mixer for 1 hour. After discontinuing the mixing, the contents of the bowl are quantitatively transferred to a 2-liter round bottom flask. The flask is installed in a Rotavapor RE11 (manufactured by Buchi) and heated to 70°C under rotation, until the blend becomes clear. After the contents of the flask become clear, vacuum provided by an aspirator pump is used to remove solvents (water and ethanol). After a substantial amount of the solvents have been removed, normally a clear, stable potassium ibuprofen liquid composition is obtained which remains clear and stable after cooling to room temperature, and after storage for 400 hours in a closed container in the absence of light at a constant temperature of 5°C. NMR analysis shows that a sample of the liquid composition produced in this manner contains about 80.6 wt% of potassium ibuprofen, about 13.4 wt% of water, and about 6.0 wt% of ethanol. The ethanol to water weight ratio is about 0.45:1.

### EXAMPLE 6

Potassium carbonate (69.1 grams) and 60 grams of deionized water are added to a Hobart stainless steel mixing bowl with stirring. After stopping the mixer, ibuprofen powder (206 grams) is added to the mixing bowl and the mixing with the Hobart mixer was resumed for 30 minutes. Then 80 grams of anhydrous ethanol is added to the mixture and the mixing is continued for another 60 minutes. After discontinuing the mixing, another 220 grams of anhydrous ethanol is used to rinse the mixing bowl and the contents of the bowl are quantitatively transferred to a 2-liter round bottom flask. The flask is installed in a Rotavapor RE11 and heated to 70°C under rotation, until the blend becomes clear. After the contents of the flask have become clear, vacuum provided by an aspirator pump is used to remove solvents (water and ethanol). After a substantial amount of the solvents have been removed, normally a clear, stable potassium ibuprofen liquid composition is obtained which remains clear and stable after cooling to room temperature, and after storage for 400 hours in a closed container in the absence of light at a constant temperature of 5°C. NMR analysis shows that a sample of the liquid composition contains about 84.2 wt% of potassium ibuprofen, about 11.5 wt% of water, and about 4.4 wt% of ethanol. The ethanol to water weight ratio is about 0.38:1.

### EXAMPLE 7

300 Grams of anhydrous ethanol and 206 grams of ibuprofen powder are added to a Hobart stainless steel mixing bowl with stirring. One mole of a 50 w/v % potassium hydroxide solution is then incrementally added to the mixing bowl under stirring. After addition of the KOH solution the mixing is continued for another 30 minutes. After discontinuing the mixing, another 250 grams of anhydrous ethanol is used to rinse the mixing bowl and the contents of the bowl are quantitatively transferred to a 2-liter round bottom flask. The contents of the flask are clear and the flask is installed in a Rotavapor RE11 and heated to 70°C under rotation. Vacuum provided by an aspirator pump is used to remove solvents (water and ethanol). After a substantial amount of the solvents have been removed, normally a clear, stable potassium ibuprofen liquid composition is obtained which remains clear and stable after cooling to room temperature, and after storage for 400 hours in a closed container in the absence of light at a constant temperature of 5°C. NMR analysis shows that a sample of the liquid composition contains about 87.0 wt% of potassium ibuprofen, about 9.5 wt% of water, and about 3.4 wt% of ethanol. The weight ratio of ethanol to water is about 0.36:1.

### EXAMPLE 8

A turbid potassium ibuprofen solution is obtained when a sample was prepared following essentially the procedures outlined in Example 7 with the exception that a higher amount of water and a lower amount of anhydrous ethanol is used. Despite the fact that a high concentration of potassium ibuprofen is contained in such a solution, it is possible to convert the turbid solution into a clear solution. Thus, with a turbid solution in which NMR analysis indicates that the turbid sample composition contained about 84.9% potassium ibuprofen, about 11.49% of water and about 3.35 wt% of ethanol, an additional amount of anhydrous ethanol is added to the turbid composition in a 2-liter flask and the flask is installed in a Rotavapor RE11 and heated to 70°C under rotation. Vacuum provided by an aspirator pump is used to remove solvents (water and ethanol). After a substantial amount of the solvents have been removed, a clear, stable potassium ibuprofen liquid composition is obtained and this liquid composition remains clear and stable after cooling to room temperature, and after storage for 400 hours in a closed container in the absence of light at a constant temperature of 5°C. NMR analysis shows that a sample of the liquid composition contains about 84.5 wt% of potassium ibuprofen, about 8.9 wt% of water, and about 4.84 wt% of ethanol. The ethanol to water weight ratio is about 0.54:1.

### EXAMPLE 9

In a Hobart stainless steel mixing bowl 206 grams of ibuprofen is dissolved in 200 grams of anhydrous ethanol. In a 500 mL beaker 69.1 grams of potassium carbonate is dissolved in 75 grams of deionized water. The contents of the beaker are then poured into the mixing bowl and mixed with the Hobart mixer for 1 hour. At the end of this mixing, the contents of the bowl are transferred to a 2-liter round bottom flask. During such transfer an additional 100 grams of ethanol is used to rinse the bowl. The flask is then installed in a Rotavapor RE11 and heated to 70°C under rotation until the blend becomes clear. After the contents of the flask have become clear, vacuum provided by an aspirator pump is used to remove solvent (water and ethanol). After a substantial amount of the solvent is removed, the contents of the bowl are poured into a glass jar. Upon cooling to ambient room temperature the potassium ibuprofen composition becomes turbid. Upon heating to 70°C the composition becomes clear and homogeneous, but again becomes turbid upon cooling to ambient room temperature. NMR analysis indicates that a sample of the liquid composition contains about 83.0 wt% of potassium ibuprofen, about 13.1 wt% of water, and about 3.9 wt% of ethanol. The ethanol to water weight ratio is slightly less than 0.3:1.

### EXAMPLE 10

The procedure of Example 6 is repeated in substantially the same manner except more solvent is removed. When cooled down to room temperature the liquid composition becomes turbid. Upon heating to 70°C the composition again becomes clear and homogeneous, but then becomes turbid upon cooling to ambient room temperature. NMR analysis shows that a sample of the liquid composition contains about 85.5 wt% of potassium ibuprofen, about 11.3 wt% of water, and about 3.2 wt% of ethanol. The ethanol to water weight ratio is about 0.28:1.

On the basis of the results achievable pursuant to Example 8, it is deemed likely that addition of some additional ethanol to the highly concentrated products in Examples 9 and 10, would result in formation of clear, stable products.

### REFERENCE EXAMPLE

This example illustrates pursuant to this invention the need for use of an alkanol specified in the description of this invention. Thus, into the stainless steel mixing bowl of a Hobart mixer are added 206 grams of ibuprofen powder and 69.1 grams of potassium carbonate. While mixing, 235.1 grams of deionized water is added to the mixture in the bowl. The blender speed is controlled to avoid overflowing the contents of the bowl. Blending is continued for 1 hour until a homogeneous paste is formed in the bowl. The paste is then transferred to a 2 liter round bottom flask. The flask is installed in a Rotavapor RE11 and heated to 70°C under rotation until the blend becomes transparent. After the contents of the flask become transparent, vacuum provided by an aspirator pump is used to remove the water. After a substantial amount of water is removed, the contents of the bowl are poured into a glass jar. Upon cooling to ambient room temperature the mixture, becomes a transparent solid. NMR analysis shows that a sample of the solid product has a potassium ibuprofen content of 58.5 wt% with the balance to 100 wt% being mostly water. Thus, in this case, there is no C₂₋₃ alkanol to water weight ratio.

In the foregoing Examples numerical values concerning the compositions of the products obtained are preceded by the word "about" because the actual experiments on which these Examples are based were conducted for convenience using anhydrous ethyl alcohol from J. T. Baker Inc., a division of Mallinckrodt Baker, Inc. The label of this ethyl alcohol indicates that the product is denatured with 5.3% (v/v) isopropyl alcohol and that the product was made from Specially Denatured Alcohol 3A which consists of 5 volumes of methanol and 100 volumes of 200 proof ethanol. Use of such denatured alcohol was deemed entirely suitable for conducting the laboratory experiments in connection with this invention. When employing anhydrous ethanol in actual commercial practice in connection with product to be used for human internal consumption, one should use a purer grade of ethyl alcohol such as pure 200 proof ethyl alcohol which contains only trace amounts, if any, of any other alcohol.

Components referred to herein by chemical name or formula, whether referred to in the singular or plural, are identified as they exist prior to coming into contact with another substance referred to by chemical name or chemical type (e.g., another component or a solvent). Also, even though the claims hereinafter may refer to substances, components and/or ingredients in the present tense (e.g., "comprises" or "is"), the reference is to the substance, component or ingredient as it existed at the time just before it was first contacted, blended or mixed with one or more other substances, components and/or ingredients in accordance with the present disclosure.

Except as may be expressly otherwise indicated, the article "a" or "an" if and as used herein is not intended to limit, and should not be construed as limiting, a claim to a single element to which the article refers. Rather, the article "a" or "an" if and as used herein is intended to cover one or more such elements, unless the text expressly indicates otherwise.

This invention is susceptible to considerable variation within the spirit and scope of the appended claims.

## Claims

1. A liquid composition containing at least 60 wt%, preferably at least 70 wt%, more preferred at least 80 wt%, and still more preferred at least 85 wt% of dissolved potassium ibuprofen, said composition being pourable at 25°C.

2. A composition as in Claim 1 wherein said composition is a clear single phase liquid composition.

3. A composition as in any of Claims 1 or 2 wherein said composition consists essentially of dissolved potassium ibuprofen, water, and at least one liquid alkanol that is miscible with water at least at 25°C.

4. A composition as in Claim 3 wherein said liquid alkanol is ethanol, 1-propanol, 2-propanol, 2-methyl-1-propanol, or a mixture of any two or more of the foregoing alkanols, preferably said liquid alkanol is ethanol, 1-propanol, 2-propanol, or a mixture of any two or all three of the foregoing alkanols, more preferred said liquid alkanol consists essentially of ethanol.

5. A concentrated pourable potassium ibuprofen liquid composition comprising (i) potassium ibuprofen, (ii) water; and (iii) methanol, ethanol, 1-propanol, 2-propanol, 1,1-dimethylethanol, or a mixture of any two or more of them, wherein the composition contains an amount of dissolved potassium ibuprofen in the range of 50 to 90 wt%, wherein the total wt% of (i), (ii), and (iii) in the composition is at least about 95 wt%, and wherein the composition is pourable at 25°C.

6. A composition as in Claim 5 wherein (iii) is ethanol, 1-propanol, or 2-propanol or a mixture of any two or all three of them, and wherein said amount of dissolved potassium ibuprofen preferably is in the range of 60 to 90 wt%.

7. A composition as in Claim 5 wherein (iii) is ethanol, and wherein the ethanol is preferably of pharmaceutically-acceptable purity.

8. A composition as in Claim 5 wherein said composition is a clear single phase liquid composition, and wherein said clear single phase liquid composition is preferably stable for at least 400 hours after preparation when stored in a closed container in the absence of light and at a temperature in the range of 5 to 70°C.

9. A composition as in Claim 7 wherein the ethanol and the water are in a weight ratio of ethanol to water in the range of 0.30:1 to 4.0:1.

10. A composition as in any of Claims 7 or 9 wherein the amount of dissolved potassium ibuprofen in said composition is in the range of 70 to 90 wt%, and the ethanol and the water are in a weight ratio of ethanol to water in the range of 0.30:1 1 to 1:1.

11. A composition as in any of Claims 7 or 9 wherein the amount of dissolved potassium ibuprofen in said composition is in the range of 80 to 90 wt%, and the ethanol and the water are in a weight ratio of ethanol to water in the range of 0.30:1 to 0.80:1.

12. A composition as in Claim 5 wherein (a) glycerol, (b) polyethylene glycol, (c) polyoxyethylene sorbitan fatty acid ester, (d) propylene glycol, or (e) a mixture of any two or more of (a), (b), (c), and (d) is included in an amount of up to about 5 wt% based on the total , weight of the composition.

13. A composition as in any of Claims 7 or 9, wherein the amount of dissolved potassium ibuprofen in said composition is in the range of 60 to 70 wt% and wherein (a) glycerol, (b) polyethylene glycol, (c) polyoxyethylene sorbitan fatty acid ester, (d) propylene glycol, or (e) a mixture of any two or more of (a), (b), (c), and (d) is included in an amount of up to about 5 wt% based on the total weight of the composition, or wherein the amount of dissolved potassium ibuprofen in said composition is in the range of 70 to 80 wt% and wherein (a) glycerol, (b) polyethylene glycol, (c) polyoxyethylene sorbitan fatty acid ester, (d) propylene glycol, or (e) a mixture of any two or more of (a), (b), (c), and (d) is included in an amount of up to about 3 wt% based on the total weight of the composition, or wherein the amount of dissolved potassium ibuprofen in said composition is in the range of 80 to 90 wt% and wherein (a) glycerol, (b) polyethylene glycol, (c) polyoxyethylene sorbitan fatty acid ester, (d) propylene glycol, or (e) a mixture of any two or more of (a), (b), (c), and (d) is included in an amount of up to about 2 wt% based on the total weight of the composition.

14. A composition as in any of Claims 7 or 9 wherein the dissolved potassium ibuprofen in said composition has an analyzable mole ratio of potassium to ibuprofen in the range of
a) 0.80:1 to 1.05:1, or
b) 0.90:1 to 1:1, or
c) 0.95:1 to 0.98:1,wherein the ethanol and the water are preferably in a weight ratio of ethanol to water in the range of 0.30:1 to 0.80:1.

15. A composition as in Claim 12 wherein said composition is a clear single phase liquid composition that is stable for at least 400 hours after preparation when stored in a closed container in the absence of light and at a temperature in the range of 5 to 70°C.

16. A composition as in either of Claims 5, 6, 7, 9 or 15 wherein except for optional presence of an excess amount of unreacted ibuprofen or unreacted potassium base, and except for optional presence of trace amounts of one or more impurities and/or manufacturing by-products, said composition contains only (i), (ii), and (iii).
